# EUROPÄISCHE PATENTSCHRIFT

(11) **EP 1 156 769 B1**
(45) Veröffentlichungstag und Bekanntmachung des Hinweises auf die Patenterteilung: **29.09.2004**
(21) Anmeldenummer: 00912465.2
(22) Anmeldetag: 16.02.2000
(51) Int. Cl.: A61J 1/00

(54) **KARTUSCHE FÜR EINE FLÜSSIGKEIT**
CARTRIDGE FOR A LIQUID
CARTOUCHE DE LIQUIDE

(30) Priorität: 23.02.1999 MY 9900627; 26.08.1999 DE 19940713
(43) Veröffentlichungstag der Anmeldung: 28.11.2001
(73) Patentinhaber: Boehringer Ingelheim International GmbH, 55216 Ingelheim am Rhein (DE)
(72) Erfinder: KLADDERS, Heinrich, D-45468 Muelheim (DE); ZIERENBERG, Bernd, D-55411 Bingen am Rhein (DE); HOCHRAINER, Dieter, D-57392 Oberkirchen (DE); EICHER, Joachim, D-44227 Dortmund (DE); HAUSMANN, Matthias, D-44287 Dortmund (DE); WUTTKE, Gilbert, D-44149 Dortmund (DE); SCHYRA, Michael, D-42111 Wuppertal (DE); FIOL, Andreas, D-81476 München (DE); GESER, Johannes, D-55218 Ingelheim (DE)
(74) Vertreter: Laudien, Dieter, Dr.
(86) Internationale Anmeldenummer: PCT/EP2000/001249
(87) Internationale Veröffentlichungsnummer: WO 2000/049988

(56) Entgegenhaltungen:
- EP-A- 0 368 112
- EP-A- 0 653 359
- WO-A-97/06842
- US-A- 5 772 080

## Beschreibung

Die Erfindung betrifft eine an ein Entnahmegerät anschließbare Kartusche für eine Flüssigkeit. Das Entnahmegerät umfaßt ein die Kartusche aufnehmendes oberes Geräteteil und ein über die angeschlossene Kartusche aufschiebbares unteres Geräteteil. Das obere Geräteteil ist mit einem Anschlußteil für die Kartusche und mit einem Entnahmestutzen zum Entnehmen der Flüssigkeit versehen.

Flüssigkeiten im Sinne der vorliegenden Erfindung können Lösungen, Suspensionen oder Emulsionen sein. Bevorzugt sind Flüssigkeiten, die eine Wirksubstanz enthalten. Wirksubstanzen können pharmakologisch aktive Wirkstoffe zur Behandlung des menschlichen oder tierischen Körpers oder Wirkstoffe zur Diagnose oder kosmetischen Anwendung sein.

Die Erfmdungs bezweckt, eine wirtschaftlich herstellbare derartige Kartusche an spezielle Forderungen anzupassen.

Es sind unterschiedliche gattungsgemäße dünnwandige Behälter für eine Flüssigkeit bekannt, die bei flüchtigen Bestandteilen der Flüssigkeit nicht diffusionsdicht sind. In diesem Fall geht ein Teil der Flüssigkeit durch Diffusion verloren, und die Konzentration von Bestandteilen der Flüssigkeit verändert sich in gegebenenfalls unzulässiger Weise. Derartige Behälter sind für eine relativ kurze Lagerzeit geeignet. Bei anderen gattungsgemäßen Behältern treten nicht annehmbare Veränderungen der Flüssigkeit durch Diffusion oder Lufteinwirkung vor und während der Verwendungsdauer auf. Besonders bei Behältern für eine Flüssigkeit, die medizinische Wirkstoffe enthält, sind verschärfte Forderungen zu erfüllen, um nicht annehmbare Qualitätseinbußen des Arzneimittels zu vermeiden.

EP-A-368 112 zeigt eine Kartusche, bei der der Stopfen, der formstabile Behälter und die Hülse mittels eines oberen Anschlußteiles, welcher einen Eintauchstutzen besitzt, zusammengehalten werden.

Damit stellt sich die Aufgabe, eine auch in großer Stückzahl wirtschaftlich herstellbare Kartusche für eine Flüssigkeit zu entwickeln, die im befüllten Zustand über lange Zeit auch unter erschwerten Bedingungen lagerfähig ist. Die Flüssigkeit soll leicht entnehmbar sein und keinen Kontakt zur Umgebung haben. Weiter soll die Kartusche an ein Entnahmegerät möglichst einfach und austauschbar anzuschließen sein; sie soll auch von ungeübten Personen zuverlässig handhabbar sein. Die Kartusche soll auch für eine Flüssigkeit, die medizinische Wirkstoffe enthält, geeignet sein und die dabei vorliegenden verschärften Bedingungen erfüllen.

Diese Aufgabe wird erfindungsgemäß gelöst durch eine Kartusche, die an ein Entnahmegerät anschließbar ist. Das Entnahmegerät umfaßt ein die Kartusche aufnehmendes oberes Geräteteil und ein über die angeschlossene Kartusche aufschiebbares unteres Geräteteil. Das obere Geräteteil ist mit einem Anschlußteil für die Kartusche und mit einem Entnahmestutzen zum Entnehmen der Flüssigkeit versehen. Die Kartusche kann als dreischaliger Behälter ausgebildet sein, der aus einer äußeren steifen Hülse, einem in der Hülse befindlichen formstabilen Behälter und einem im formstabilen Behälter angeordneten kollabierbaren Beutel besteht, der die Flüssigkeit enthält. Die steife Hülse kann einen Boden haben, der mit einer Öffnung versehen sein kann. Der formstabile Behälter kann ebenfalls eine Öffnung haben und mit einem Stopfen verschlossen sein, der mit einem Eintauchstutzen versehen ist. Der Stopfen kann eine dicht schließende zentrierte Führung für den Entnahmestutzen bilden. Der Stopfen kann mit dem formstabilen Behälter durch die steife Hülse unlösbar verbunden sein. Die Kartusche kann mit dem Anschlußteil am oberen Geräteteil des Entnahmegerätes lösbar verbunden sein.

Die Verbindung zwischen Kartusche und Anschlußteil kann als Steckverbindung, als Schraubverbindung oder als Bajonettverbindung ausgeführt sein. Diese Verbindung kann bevorzugt eine lösbare Verbindung sein. Gegebenenfalls kann sie als schwer lösbare oder als unlösbare Verbindung ausgeführt sein.

Der Stopfen kann bevorzugt aus einem thermoplastischen Kunststoff bestehen und kann mit dem formstabilen Behälter kraft- und formschlüssig mittels einer Schnappverbindung verbunden sein. Weiter kann der Stopfen mit dem formstabilen Behälter aus thermoplastischem Kunststoff stoffschlüssig unlösbar verschweißt sein. Der Stopfen kann im Eintauchstutzen mit einer trichterförmigen zentrierten Führung für den Entnahmestutzen versehen sein. Die Führung kann mit Führungsrippen versehen sein. Der Stopfen kann den Entnahmestutzen dicht umschließen und als Preßpassung für den Entnahmestutzen ausgebildet sein. Es kann zweckmäßig sein, das dem Innenraum des kollabierbaren Beutels zugewandte Ende des Eintauchstutzens mit einer Membran zu verschließen, die gegen die Achse des Eintauchstutzens geneigt angebracht sein kann, und die beim Einführen des Entnahmestutzens in die Kartusche durchstochen werden kann. Diese Membran verhindert das Austreten der Flüssigkeit in den Eintauchstutzen während der Lagerung der Kartusche.

Zwischen der Oberkante des formstabilen Behälters und der Innenseite des Stopfens kann eine Dichtscheibe liegen, die mit Dichtwülsten versehen sein kann. Die Innenseite des Stopfens kann mit einer Dichtlippe oder mit mehreren Dichtlippen versehen sein, die in die Dichtscheibe auf der Oberkante des formstabilen Behälters eingedrückt sind.

Die steife Hülse ist - abgesehen von ihrer Öffnung - diffusionsdicht gegen Gase und Flüssigkeiten. Diese Hülse kann eine einstückige tiefgezogene Metallhülse, bevorzugt aus Aluminium, sein. Ferner kann die steife Hülse zweistückig ausgebildet sein; dann werden die beiden Hülsenteile über Dichtelemente, durch Schweißen oder durch Kleben miteinander verbunden und gegeneinander abgedichtet. Weiter kann die steife Hülse aus einem Kunststoff bestehen, bevorzugt aus einem Thermoplasten.

Die steife Hülse kann am Rand ihres Bodens eine vorspringende Wulst haben. Der Boden kann mit einer Vertiefung versehen sein, die als nach innen gestülpter Bereich des Bodens zentral angeordnet sein kann.

Die steife Hülse hat bevorzugt im Mittelpunkt des Bodens eine Öffnung, die als Bohrung ausgeführt sein kann. Zusätzlich kann in der Vertiefung des Hülsenbodens ein bevorzugt aus Kunststoff bestehender Einsatz angebracht sein, der eine Öffnung in Form einer Mikroöffnung enthält, die mit der Öffnung im Boden der steifen Hülse in Verbindung steht. Innerhalb des Einsatzes kann vor der Mikroöffnung ein Filter angebracht sein.

Die Öffnung im Boden der steifen Hülse kann bei kreisförmigem Querschnitt einen Durchmesser von 0,1 mm bis 5 mm haben. Die Mikroöffnung im Einsatz hat bei kreisförmigem Querschnitt einen Durchmesser von 10 µm bis 500 µm und eine Länge von 100 µm bis 5000 µm. Die Mikroöffnung ermöglicht, die Zeit für den Druckausgleich zwischen dem Innenraum der Kartusche und ihrer Umgebung auf einen gewünschten Wert einzustellen.

Die steife Hülse aus Metall kann in der Nähe ihres offenen Endes mit einer umlaufenden Sicke versehen sein, die den Stopfen kraft- und formschlüssig umschließt. Die steife Hülse aus thermoplastischem Kunststoff kann mit dem Stopfen stoffschlüssig verschweißt sein. Weiter kann die steife Hülse in ihrem oberen Teil mit einer umlaufenden nach außen offenen Rille versehen sein, die die Unterkante des Stopfens umschließt. Die steife Hülse kann an ihrem offenen Ende eine Umbördelung haben, die die Oberkante des Stopfens umschließt.

Die steife Hülse kann mit mehreren in das Innere der Hülse ragenden Nocken versehen sein, die den formstabilen Behälter innerhalb der steifen Hülse reibschlüssig abstützen. Bevorzugt werden drei Nocken im mittleren bis unteren Bereich der steifen Hülse angebracht, die in einer Ebene liegen, die senkrecht auf der Hülsenachse steht. Bei einer steifen Hülse aus Metall können diese Nocken beim Bördeln des offenen Endes der steifen Hülse angebracht werden.

Die Kartusche kann im Bereich des Stopfens mit einer - gegebenenfalls diffusionsdichten - Siegelfolie versiegelt sein, die das offene Ende des Eintauchstutzens verschließt. Die Außenseite des Hülsenbodens kann ebenfalls mit einer - gegebenenfalls diffusionsdichten - Siegelfolie versiegelt sein, die die Bodenöffnung oder den Einsatz im Hülsenboden abdeckt. Beide Siegelfolien verhindern das Eindringen von Schmutz in die darunter liegenden Öffnungen und die Diffusion von Bestandteilen der Flüssigkeit während der Lagerzeit der Kartusche. Beide Siegelfolien werden erst unmittelbar vor dem ersten bestimmungsgemäßen Gebrauch der Kartusche abgelöst oder durchstochen.

Die lösbare Verbindung zwischen Kartusche und Anschlußteil des Entnahmegerätes kann eine Steckverbindung sein, bei der das Anschlußteil mit mehreren Schnapphaken versehen ist, die in die umlaufende Rille im Oberteil der steifen Hülse eingreifen, nachdem die Kartusche in das Entnahmegerät eingeschoben worden ist. Die Steckverbindung läßt sich vorteilhaft auch für andere gattungsgemäße Behälter verwenden, wobei der formstabile Behälter oder der Stopfen eine Rille enthält, in die die Schnapphaken eingreifen.

Schnapphaken aus Kunststoff können mit einem metallischen Federelement versehen sein, das die Federeigenschaft der Schnapphaken über lange Zeit und bei erhöhter Temperatur aufrechterhält.

Im mittleren Bereich der Siegelfolie auf der Außenseite des Hülsenbodens kann ein von der Siegelfolie bedeckter freier Raum vorhanden sein. Auf der Innenseite des Bodens des unteren Geräteteils kann eine starre oder federnde Anstechvorrichtung angebracht sein, die die auf der Unterseite des Hülsenbodens angebrachte Siegelfolie vor dem ersten Entnehmen eines Teils der Flüssigkeit aus der Kartusche durchsticht. Damit wird die Öffnung im Hülsenboden oder die Mikroöffnung im Einsatz freigegeben und der Eintritt von Luft in den Innenraum der Kartusche ermöglicht.

Zum Herausziehen einer verbrauchten Kartusche aus dem Entnahmegerät kann gegebenenfalls eine Abziehhilfe benutzt werden, die unter die Wulst am Boden der steifen Hülse geschoben wird, wodurch das Herausziehen der Kartusche erleichtert wird.

Vor dem Anschließen der Kartusche an das Entnahmegerät werden die Siegelfolien auf dem Stopfen und auf dem Boden der Kartusche abgelöst, oder diese Siegelfolien werden beim Anschließen der Kartusche an das Entnahmegerät durchstochen. Ein zwischen dem Innern der Kartusche und der Umgebung der Kartusche gegebenenfalls vorhandener Druckunterschied gleicht sich durch die Öffnung im Boden der steifen Hülse aus.

Beim Entnehmen einer Flüssigkeitsmenge aus der an das Entnahmegerät angeschlossenen Kartusche zieht sich der kollabierbare Beutel zusammen, und sein Volumen verkleinert sich um das Volumen der entnommenen Flüssigkeitsmenge. Dadurch entsteht ein Unterdruck im Gasraum der Kartusche (das ist der freie Raum zwischen der Außenseite des kollabierbaren Beutels und der Innenseite der steifen Hülse) im Vergleich zum Druck in der Umgebung der Kartusche. Dieser Druckunterschied gleicht sich in relativ kurzer Zeit aus, wenn die steife Hülse dünnwandig ist und mit einer Bohrung im Bereich von 0,1 mm bis 5 mm versehen ist.

Falls die Kartusche im Boden der steifen Hülse mit einem Einsatz versehen ist, der eine Mikroöffnung enthält, läßt sich die Zeit für den Druckausgleich zwischen dem Gasraum in der Kartusche und der Umgebung einstellen. Zum Beispiel läßt sich bei einem Volumen von 3 Millilitern des Gasraums in der Kartusche und einem Druckunterschied von 20 hPa (20 mbar) zwischen der Umgebung der Kartusche und dem Gasraum in der Kartusche bei einer kreisförmigen Mikroöffnung mit einer Länge von 200 µm und einem Durchmesser von 80 µm bis 50 µm eine Halbwertszeit für den Druckausgleich von 2 Stunden bis 13 Stunden erreichen.

Bei einer Druckausgleichszeit, die an den bestimmungsgemäßen zeitlichen Abstand zwischen zwei Entnahmevorgängen aus der Kartusche angepaßt ist, wird die Diffusion von Bestandteilen der Flüssigkeit aus dem kollabierbaren Beutel erschwert.

Die erfindungsgemäße Kartusche kann beispielsweise eine Länge von 55 mm und einen Durchmesser von 17 mm haben. Der Stopfen kann einen Eintauchstutzen haben, dessen Innendurchmesser auf einem Entnahmestutzen mit einem Außendurchmesser von 2 mm einen festen Preßsitz ergibt.

Die erfindungsgemäße Kartusche kann in einem Zerstäuber eingesetzt werden, wie er in den Figuren 6a und 6b in WO - 97/12687 dargestellt ist. Die Kartusche (1) der vorliegenden Erfindung entspricht dem Vorratsbehälter (71) in den Figuren 6a und 6b, das als Anschlußteil (2) ausgebildete Sprungstück des Sperrspannwerkes entspricht dem Sprungstück (56), und das untere Geräteteil (3) entspricht dem unteren Gehäuseteil (70).

Die Kartusche kann eine wässrige oder alkoholische Flüssigkeit enthalten.

Bei einer wässrigen Flüssigkeit können der Formstabile Behälter und der Stopfen aus Polypropylen bestehen. Der kollabierbare Beutel kann aus Polyethylen bestehen. Die steife Hülse kann aus Kunststoff, bevorzugt aus Polypropylen bestehen. Die Öffnung im Boden der steifen Hülse kann eine Bohrung sein. Der Stopfen für den formstabilen Behälter kann mit einem Eintauchstutzen versehen sein, der an seinem dem Innenraum zugewandten Ende mit einer gegen die Achse des Eintauchstutzens geneigten Membran verschlossen sein kann. Der Eintauchstutzen kann eine Preßpassung für den Entnahmestutzen enthalten. Der Stopfen kann mit dem formstabilen Behälter durch eine Schnappverbindung verbunden sein. Die lösbare Steckverbindung zwischen Kartusche und Anschlußteil des Entnahmegerätes kann eine Schnappverbindung sein, bei der die Schnapphaken im Anschlußteil des Entnahmegerätes in die umlaufende Rille im oberen Bereich der Kartusche eingreifen. Die Innenseite des Bodens des aufgesteckten unteren Geräteteils kann mit einer federnden Anstechvorrichtung für die Siegelfolie auf der Unterseite des Hülsenbodens versehen sein.

Bei einer alkoholischen Flüssigkeit können der formstabile Behälter und der Stopfen aus Polypropylen bestehen. Der kollabierbare Beutel kann aus Polyethylen bestehen. Die steife Hülse kann aus Metall, bevorzugt aus Aluminium, bestehen. In der Vertiefung im Boden der steifen Hülse kann eine Einsatz mit einer Mikroöffnung angebracht sein, die mit der Bohrung im Hülsenboden in Verbindung steht. Der mit einem Eintauchstutzen versehene Stopfen für den formstabilen Behälter kann an seinem dem Innenraum zugewandten Ende mit einer gegen die Achse des Eintauchstutzens geneigten Membran verschlossen sein. Der Eintauchstutzen kann mit einer als Preßpassung ausgebildeten dicht schließenden zentrierten Führung für den Entnahmestutzen versehen sein. Der Stopfen kann mit dem formstabilen Behälter mittels der steifen Hülse unlösbar verbunden sein. Die lösbare Steckverbindung zwischen Kartusche und Anschlußteil des Entnahmegerätes kann eine Schnappverbindung sein, bei der die Schnapphaken im Anschlußteil des Eninahmegerätes in die umlaufende Rille im oberen Bereich der Kartusche eingreifen. Die Innenseite des Bodens des aufgesteckten unteren Geräteteils kann mit einer federnden Anstechvorrichtung für die Siegelfolie auf der Unterseite des Hülsenbodens versehen sein.

Die erfindungsgemäße Kartusche kann mit einer medizinischen Flüssigkeit gefüllt sein, die zum Beispiel einen pharmakologisch aktiven Wirkstoff sowie beispielsweise Wasser, Ethanol oder deren Mischungen enthält.

In WO - 98/27959 werden stabilisierte wässrige Arzneimittelzubereitungen zum Herstellen von treibgasfreien Aerosolen für die Inhalation beschrieben. Auf die dort beanspruchten und in den Beispielen angegebenen Formulierungen wird Bezug genommen.

Geeignete Arzneimittel-Zubereitungen in ethanolischer Lösung sind beispielsweise in WO - 97/01329 angegeben, insbesondere wird auf die dort genannten Wirkstoffe (siehe dort die Seiten 2 und 3) sowie die dort beanspruchten stabilisierten Formulierungen Bezug genommen.

Als medizinische Wirkstoffe können Berotec (Fenoterol-Hydrebromid; 1-(3,5-dihydroxyphenyl)-2-[[1-(4-hydroxy-benzyl)-ethyl]-amino]-ethanol-hydrobromid), Atrovent (Ipratropiumbromid), Berodual (Kombination aus Fenoterol-Hydrobromid und Ipratropiumbromid), Salbutamol, Salbutamolsulfat, Combivent, Oxivent (Oxitropium-bromid), Ba 679 (Tiotropiumbromid), BEA 2108 (Di-(2-thienyl)-glykolsäuretropenolester), Flunisolid, Budesonid, Beclomethason und andere verwendet werden.

Die erfindungsgemäße Kartusche hat folgende Vorteile:
- Die Konzentration der in der versiegelten Kartusche enthaltenen medizinischen Flüssigkeit verändert sich auch bei mehrjähriger Lagerzeit und bei erhöhter Lagertemperatur nur in einem tolerierbaren Ausmaß.
- Der Inhalt der Kartusche ist gegen Verunreinigungen sehr wirksam geschützt.
- Die in das Entnahmegerät eingesteckte Kartusche kann über mehrere Monate verwendet werden.
- Die Halbwertszeit für den Druckausgleich ist durch die Form der Mikroöffnung einstellbar.
- Die Kartusche kann in medizinisch einwandfreier Form hergestellt werden.
- Der unveränderte Originalzustand der Kartusche und ihres Inhalts läßt sich innerhalb der angegebenen Verwendbarkeitsdauer an den unversehrten Siegelfolien einwandfrei erkennen.
- Die Kartusche hat eine große Funktionssicherheit; Fehlbedienungen werden unterbunden.
- Die Kartusche wird im Entnahmegerät sicher gehalten.
- Die fest verschlossene Kartusche ist kindersicher und erschwert Manipulationen am Inhalt.
- Die leere Kartusche läßt sich wegen der Wulst am Boden der steifen Hülse auf einfache Weise aus dem Entnahmegerät herausziehen.

Die erfindungsgemäße Kartusche, die eine medizinische Flüssigkeit enthält, kann zum Erzeugen eines inhalierbaren Aerosols mittels eines Zerstäubers verwendet werden. Das Aerosol kann zum Behandeln von Krankheiten dienen.

Die erfindungsgemäße Kartusche wird an Hand der Figuren weiter erläutert.

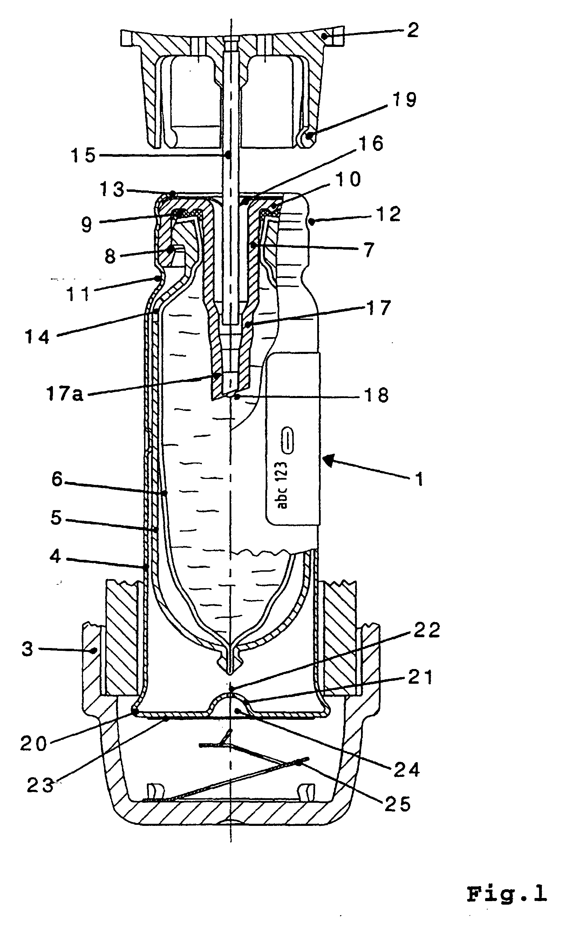

Figur 1 zeigt die Kartusche (1), das Anschlußteil (2) des Entnahmegerätes und das untere Geräteteil (3), das über die angeschlossene Kartusche geschoben wird. Die steife Hülse (4) enthält den formstabilen Behälter (5) mit dem kollabierbaren Beutel (6). Der formstabile Behälter ist mit dem Stopfen (7) und der Schnappverbindung (8) verschlossen. Zwischen der Oberkante des formstabilen Behälters und der Unterseite des Stopfens liegt die Dichtscheibe (9), in die die Dichtlippe (10) eingedrückt ist. Die steife Hülse enthält die umlaufende Rille (11), an der die Unterkante des Stopfens anliegt. Die umlaufende Sicke (12) in der steifen Hülse ist in den Stopfen eingedrückt. Der Stopfen wird an seiner Oberseite von der Umbördelung (13) des oberen Endes der steifen Hülse gehalten. Im formstabilen Behälter (5) ist die Öffnung (14) angebracht.

Das Anschlußteil des Entnahmegerätes enthält den Entnahmestutzen (15), der die Siegelfolie (16) auf der Oberseite des Stopfens bereits durchstochen hat und in den Eintauchstutzen (17) eingedrungen ist. Beim weiteren Aufschieben der Kartusche auf den Entnahmestutzen durchdringt dieser den Bereich (17a) der Preßpassung und durchstößt die geneigte Membran (18) am Ende des Eintauchstutzens. Die Schnapphaken (19) am Anschlußteil (2) des Entnahmegerätes greifen von außen in die umlaufende Rille (11) in der steifen Hülse ein.

Die steife Hülse ist an ihrem Bodenrand mit der vorspringenden Wulst (20) versehen. In der Mitte der Vertiefung in Form des nach innen gestülpten Bereiches (21) befindet sich die Öffnung (22) in der steifen Hülse. Der Boden der steifen Hülse ist mit der Siegelfolie (23) bedeckt. Zwischen der Siegelfolie und dem nach innen gewölbten Bereich befindet sich der freie Raum (24). Auf der Innenseite des unteren Geräteteils (3) ist die federnde Anstechvorrichtung (25) angebracht.

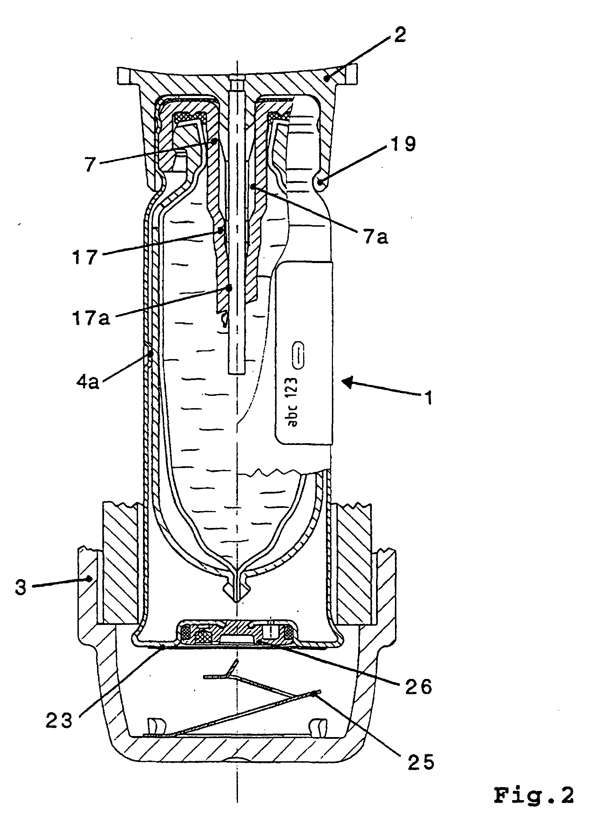

Figur 2 zeigt die auf den Entnahmestutzen vollständig aufgeschobene und an das Anschlußteil (2) angeschlossene Kartusche (1). Die Membran am Ende des Eintauchstutzens ist durchstochen, und die Schnapphaken (19) greifen in die umlaufende Rille am oberen Ende der steifen Hülse ein. Der Stopfen (7) ist mit Führungsrippen (7a) versehen. Im Bereich (17a) besteht die Preßpassung zwischen dem Entnahmestutzen und dem Stopfen. In der Vertiefung in Form des nach innen gestülpten Bereichs des Hülsenbodens ist der Einsatz (26) angebracht.

Die steife Hülse (4) ist in ihrem mittleren Bereich mit Nocken (4 a) versehen, die in einer Ebene liegen, die senkrecht zur Hülsenachse steht. Diese Nocken stützen den formstabilen Behälter.

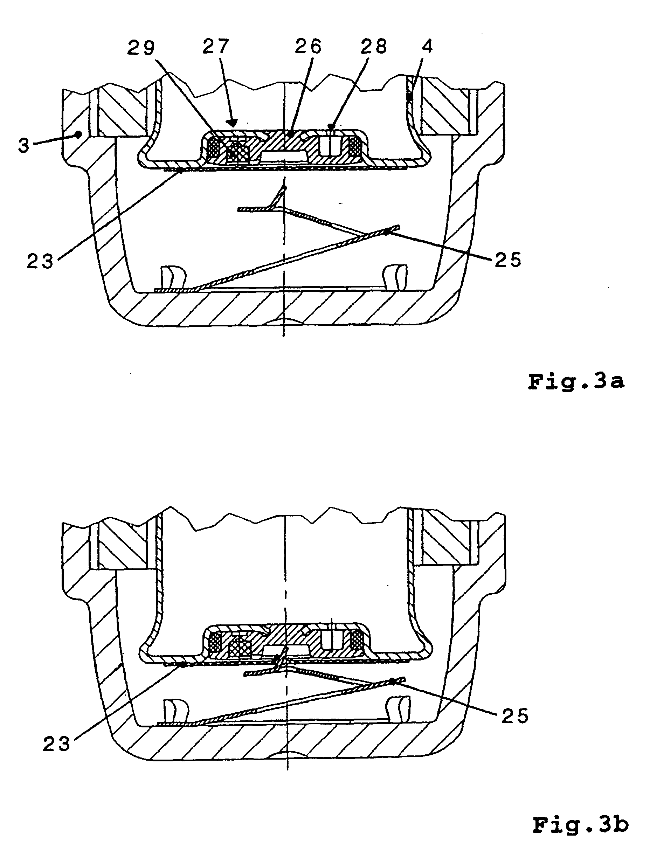

Figur 3 a zeigt das Ende der steifen Hülse (4), das Ende des unteren Geräteteils (3) und den Einsatz (26) in vergößerter Darstellung. Der Einsatz enthält die Mikroöffnung (27), die mit der Öffnung (28) im Boden der steifen Hülse in Verbindung steht. Vor der Mikroöffnung befindet sich das Filter (29). Der Boden der steifen Hülse ist mit der Siegelfolie (23) bedeckt.

In Figur 3 b ist der Zustand dargestellt, bei dem die Anstechvorrichtung (25) die Siegelfolie (23) durchstochen hat.

In Figur 4 a ist ein Querschnitt durch die Abziehhilfe (30) dargestellt, die auf die Kartusche (1) aufgesteckt ist und die steife Hülse hinter der Wulst (20) eingedrückt hat. Die Kartusche ist in die Abziehhilfe eingeklemmt und kann mittels der Abziehhilfe um die Achse der Kartusche gedreht und aus dem Entnahmegerät herausgezogen werden.

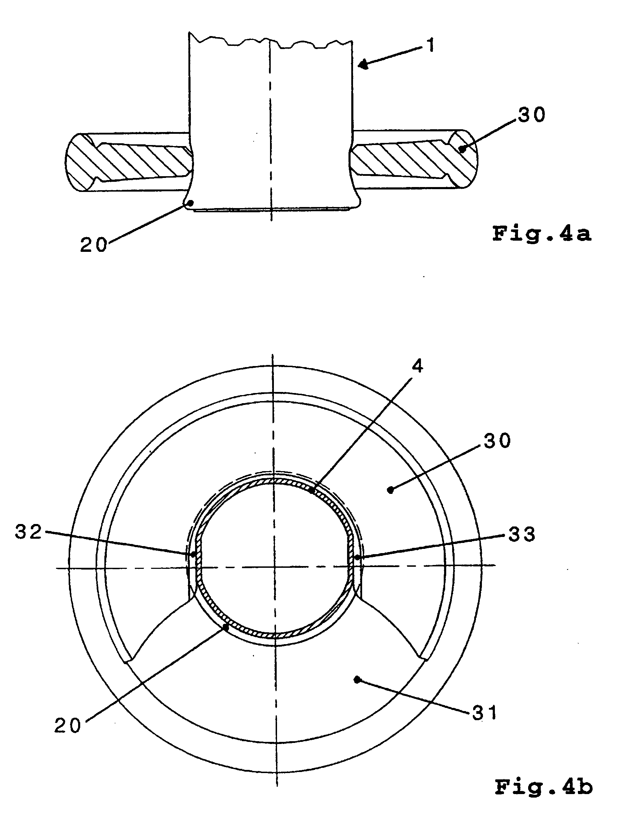

Figur 4 b zeigt die aufgesteckte Abziehhilfe (30) in der Ansicht von der Oberseite sowie die steife Hülse (4) im Querschnitt. Die Abziehhilfe enthält eine Aussparung mit einem zentral angeordneten Bereich, dessen Durchmesser mit dem Außendurchmesser der steifen Hülse (4) im wesentlichen übereinstimmt und der kleiner ist als der Durchmesser der Wulst (20). Der zentrale Bereich der Aussparung geht in eine erweiterte Aussparung (31) über, damit die Abziehhilfe auf das Ende der Kartusche leicht aufgesteckt werden kann. Der Durchmesser des zentralen Bereiches der Aussparung ist an zwei einander diametral gegenüberliegenden Stellen (32) und (33) verkleinert, und der Kreisbogen der Aussparung ist abgeflacht. An diesen Stellen wird die steife Hülse der Kartusche beim Aufschieben der Abziehhilfe zusammengedrückt und eine stramme Verbindung der Abziehhilfe mit der Kartusche hergestellt.

## Patentansprüche

1. Kartusche für eine Flüssigkeit, die an ein Entnahmegerät anschließbar ist, das ein die Kartusche aufnehmendes oberes Geräteteil und ein über die angeschlossene Kartusche aufschiebbares unteres Geräteteil umfasst, und das obere Geräteteil mit einem Anschlussteil für die Kartusche und mit einem Entnahmestutzen zum Entnehmen der Flüssigkeit versehen ist, wobei
• die Kartusche (1) ein dreischaliger Behälter ist und aus einer äußeren steifen Hülse (4), einem in der Hülse befindlichen formstabilen Behälter (5) und einem im formstabilen Behälter angeordneten kollabierbaren Beutel (6) besteht, der die Flüssigkeit enthält, und
• die steife Hülse einen Boden hat, der mit einer Öffnung (22) versehen ist, und
• der formstabile Behälter mit einer Öffnung (14) versehen ist und mit einem Stopfen (7) verschlossen ist, und
• der Stopfen mit einem Eintauchstutzen (17) versehen ist, der eine dicht schließende zentrierte Führung für den Entnahmestutzen bildet, und
• der Stopfen mit dem formstabilen Behälter durch die Hülse (4) unlösbar verbunden ist.

2. Kartusche nach Anspruch 1, wobei
• die Kartusche (1) mit dem Anschlußteil (2) mittels einer Steckverbindung lösbar verbunden ist.

3. Kartusche nach Anspruch 1, wobei
• die Kartusche (1) mit dem Anschlussteil (2) mittels einer Schraubverbindung oder einer Bajonettverbindung lösbar verbunden ist.

4. Kartusche nach den Ansprüchen 1 bis 3, wobei
• der Stopfen (7), bevorzugt aus einem thermoplastischen Kunststoff, mit dem formstabilen Behälter (5) kraft- und formschlüssig mittels einer Schnappverbindung (8) verbunden ist.

5. Kartusche nach den Ansprüchen 1 bis 3, wobei
• der Stopfen (7) aus einem thermoplastischem Kunststoff mit dem formstabile Behälter (5) aus einem thermoplastischen Kunststoff stoffschlüssig mittels einer Schweißverbindung unlösbar verbunden ist.

6. Kartusche nach den Ansprüchen 1 bis 5, wobei
• der Stopfen (7) im Eintauchstutzen (17) mit einer trichterförmigen zentrierten Führung versehen ist, die gegebenenfalls Führungsrippen (7a) enthält.

7. Kartusche nach den Ansprüchen 1 bis 6, wobei
• der Stopfen (7) als dicht abschließende Führung für den Entnahmestutzen mit einer Presspassung (17a) versehen ist.

8. Kartusche nach den Ansprüchen 1 bis 7, wobei
• der Stopfen (7) am Ende des Eintauchstutzens mit einer Membran (18) versehen ist, die bevorzugt geneigt gegen die Achse des Eintauchstutzens angebracht ist.

9. Kartusche nach den Ansprüchen 1 bis 4 oder 6 bis 8, wobei
• zwischen der Oberkante des formstabilen Behälters und der Innenseite des Stopfens eine Dichtscheibe (9) liegt, die gegebenenfalls mit Dichtwülsten versehen ist.

10. Kartusche nach den Ansprüchen 1 bis 9, wobei
• die Innenseite des Stopfens mit einer Dichtlippe (10) oder mit mehreren Dichtlippen versehen ist.

11. Kartusche nach den Ansprüchen 1 bis 10, wobei
• die steife Hülse (4) - abgesehen von ihrer Öffnung - diffusionsdicht ist.

12. Kartusche nach den Ansprüchen 1 bis 11, wobei
• die steife Hülse (4) mit mehreren nach innen ragenden Nocken (4 a) versehen ist.

13. Kartusche nach den Ansprüchen 1 bis 12, wobei
• die steife Hülse (4) einstückig ausgebildet ist.

14. Kartusche nach den Ansprüchen 1 bis 12, wobei
• die steife Hülse zweistückig ausgebildet ist.

15. Kartusche nach den Ansprüchen 1 bis 14, wobei
• die steife Hülse (4) eine tiefgezogene Metallhülse ist, bevorzugt aus Aluminium.

16. Kartusche nach den Ansprüchen 1 bis 14, wobei
• die steife Hülse (4) aus einem Kunststoff besteht, bevorzugt aus einem Thermoplasten.

17. Kartusche nach den Ansprüchen 1 bis 16, wobei
• die steife Hülse (4) an ihrem Boden mit einer vorspringenden Wulst (20) versehen ist.

18. Kartusche nach den Ansprüchen 1 bis 17, wobei
• die steife Hülse (4) in ihrem Boden ein Vertiefung hat.

19. Kartusche nach Anspruch 18, wobei
• die steife Hülse (4) innerhalb der Vertiefung mit einer Öffnung (22) versehen ist, deren Durchmesser bei kreisförmigem Querschnitt von 0,1 Millimeter bis 5 Millimeter beträgt.

20. Kartusche nach den Anspruch 18, wobei
• die steife Hülse (4) innerhalb der Vertiefung mit einem Einsatz (26) versehen ist, der eine Öffnung, bevorzugt in Form einer Mikroöffnung (27), enthält, die mit der Öffnung (28) im Boden der steifen Hülse in Verbindung steht, und die einen Durchmesser bei kreisförmigem Querschnitt von 10 µm bis 500 µm und eine Länge von 100 µm bis 500 µm hat.

21. Kartusche nach den Ansprüchen 1 bis 20, wobei
• der Einsatz (26) mit einem Filter (29) vor der Mikroöffnung (27) versehen ist.

22. Kartusche nach den Ansprüchen 1 bis 21, wobei
• die steife Hülse (4) aus Metall mit mindestens einer umlaufenden Sicke (12) versehen ist, die den Stopfen (4) kraft- und formschlüssig umschließt.

23. Kartusche nach den Ansprüchen 1 bis 21, wobei
• die steife Hülse (4) aus thermoplastischem Kunststoff mit dem Stopfen (7) mittels einer Schweißverbindung stoffschlüssig verbunden ist.

24. Kartusche nach den Ansprüchen 1 bis 23, wobei
• die steife Hülse (4) in ihrem oberen Teil mit einer umlaufenden Rille (11) versehen ist, die die Unterkante des Stopfens (7) umschließt.

25. Kartusche nach den Ansprüchen 1 bis 24, wobei
• die steife Hülse (4) in ihrem oberen Teil mit einer Umbördelung (13) versehen ist, die die Oberkante des Stopfens (7) umschließt.

26. Kartusche nach den Ansprüchen 1 bis 25, wobei
• die Kartusche im Bereich des Stopfens (7) mit einer - gegebenenfalls diffusionsdichten - Siegelfolie (16) versiegelt ist.

27. Kartusche nach den Ansprüchen 1 bis 26, wobei
• die steife Hülse (4) auf der Außenseite ihres Bodens mit einer- gegebenenfalls diffusionsdichten - Siegelfolie (23) versiegelt ist.

28. Kartusche nach den Ansprüchen 1 bis 27, wobei
• im mittleren Bereich der Siegelfolie (23) auf der Außenseite des Bodens der steifen Hülse (4) ein von der Siegelfolie bedeckter freier Raum (24) vorhanden ist.

29. Kartusche nach den Ansprüchen 1 bis 28,
• die mittels einer hinter die vorspringende Wulst (20) aufsteckbaren Abziehhilfe (30) aus dem Entnahmegerät herausziehbar ist.

30. Kartusche nach Anspruch 1, wobei
• die Kartusche mit dem Anschlussteil (2) mit dem oberen Geräteteil lösbar verbunden ist, und
• Anschlussteil (2) mit Schnapphaken (19) versehen ist, die in die umlaufende Rille (11) im Oberteil der steifen Hülse (4) eingreifen, nachdem die Kartusche in das Entnahmegerät eingeschoben worden ist.

31. Kartusche nach Anspruch 1, wobei
• die Kartusche mit dem Anschlussteil (2) mit dem oberen Geräteteil lösbar verbunden ist, und
• das Anschlussteil (2) mit Schnapphaken (19) versehen ist, die in eine umlaufende Rille im Stopfen (7) oder im formstabilen Behälter (5) eingreifen, nachdem der formstabile Behälter in das Entnahmegerät eingeschoben worden ist.

32. Kartusche nach Anspruch 1 für eine wässrige Flüssigkeit, bestehend aus einem formstabilen Behälter (5) aus Polypropylen mit einem Stopfen (7) aus Polypropylen und mit einem im Behälter (5) befindlichen kollabierbaren Beutel (6) aus Polyethylen, wobei
• die steife Hülse (4) aus Kunststoff, bevorzugt aus Polypropylen, besteht, und
• die Öffnung (22) im Boden der steifen Hülse (4) eine Bohrung ist, und
• der Stopfen (7) für den formstabilen Behälter (5) mit einem Eintauchstutzen (17) versehen ist, der an seinem Ende mit einer gegen die Achse des Eintauchstutzens geneigten Membran (18) verschlossen ist, und der eine als Presspassung ausgebildete dicht abschließende zentrierte Führung für den Entnahmestutzen (15) bildet, und der mit dem formstabilen Behälter durch eine Schnappverbindung verbunden ist, und
• die lösbare Steckverbindung zwischen Kartusche (1) und Anschlußteil (2) des Entnahmegerätes als Schnappverbindung ausgebildet ist, bei der Schnapphaken (19) im Anschlussteil (2) des oberen Geräteteils vorhanden sind, die in die umlaufende Rille (11) im oberen Bereich der Kartusche (1) eingreifen, und
• die Innenseite des Bodens des aufsteckbaren Geräteteils (3) mit der federnden Anstechvorrichtung (25) für die Siegelfolie (23) auf der Unterseite des Hülsenbodens versehen ist.

33. Kartusche nach Anspruch 1 für eine alkoholische Flüssigkeit, bestehend aus einem form stabilen Behälter (5) aus Polypropylen mit einem Stopfen aus Polypropylen und mit einem im Behälter (5) befindlichen kollabierbaren Beutel (6) aus Polyethylen, wobei
• die steife Hülse (4) aus Metall, bevorzugt aus Aluminium, besteht, und
• der mit einem Eintauchstutzen (17) versehene Stopfen (7) für den formstabilen Behälter (5) an seinem Ende mit einer gegen die Achse des Eintauchstutzens geneigten Membran (18) verschlossen ist, und
• der Stopfen (7) mit einer als Presspassung ausgebildeten dicht schließenden zentrierte Führung für den Entnahmestutzen (15) versehen ist, und
• der Stopfen (7) mit dem formstabilen Behälter (5) durch die Hülse (4) unlösbar verbunden ist, und
• die lösbare Steckverbindung zwischen Kartusche (1) und Anschlußteil (2) des Entnahmegerätes als Schnappverbindung ausgebildet ist, bei der Schnapphaken (19) im Anschlussteil (2) des Entnahmegerätes vorhanden sind, die in die umlaufende Rille (11) im oberen Bereich der Kartusche eingreifen, und
• im Boden der steifen Hülse (4) ein Einsatz (26) mit einer Mikroöffnung (27) vorhanden ist, und die Mikroöffnung mit einer Bohrung (28) in der steifen Hülse (4) in Verbindung steht, und
• die Innenseite des Bodens des aufsteckbaren Geräteteils (3) mit der federnden Anstechvorrichtung (25) für die Siegelfolie (23) auf der Unterseite des Hülsenbodens versehen ist.

34. Kartusche nach den Ansprüchen 1, 32 und 33, wobei
• die Flüssigkeit einen pharmakologisch aktiven Wirkstoff enthält.

35. Kartusche nach den Ansprüchen 1 und 32 bis 34 für eine medizinische Flüssigkeit, wobei
• die medizinische Flüssigkeit einen oder mehrere der Wirkstoffe Berotec (Fenoterol Hydrobromid; 1-(3,5-dihydroxy-phenyl)-2-[[1-(4-hydroxy-benzyl)-ethyl]-amino] ethanol-hydrobromid), Atrovent (Ipratropiumbromid), Berodual (Kombination aus Fenoterol-Hydrobromid und Ipratropium-bromid), Salbutamol, Salbutamolsulfat, Combivent, Oxivent (Oxitropiumbromid), Ba 679 (Tiotropiumbromid), BEA 2108 (Di-(2-thienyl)-glykolsäuretropenolester), Flunisolid, Budesonid, Beclomethason enthält.

36. Verwendung der Kartusche nach den Ansprüchen 1 bis 35, enthaltend eine medizinische Flüssigkeit, zur Erzeugung eines Aerosols mittels eines Zerstäubers.

37. Verwendung der Kartusche nach den Ansprüchen 1 bis 35, enthaltend eine medizinische Flüssigkeit, zur Erzeugung eines inhalierbaren Aerosols zur Behandlung von Krankheiten.

38. Verwendung einer Kartusche nach Anspruch 1 in einem Entnahmegerät, das ein die Kartusche aufnehmendes oberes Geräteteil und ein über die angeschlossene Kartusche aufschiebbares unteres Geräteteil (3) umfasst und das obere Geräteteil mit einem Anschlussteil für die Kartusche und mit einem Entnahmestutzen (15) zum Entnehmen der Flüssigkeit versehen ist, wobei die Innenseite des Bodens des unteren Geräteteils (3) mit einer Anstechvorrichtung für die auf der Unterseite des Hülsenbodens angebrachte Siegelfolie (23) versehen ist.

39. Entnahmegerät umfassend
- eine Kartusche nach einem der Ansprüche 1 bis 35,
- ein oberes Geräteteil, das die Kartusche aufnimmt und
- ein über die angeschlossene Kartusche aufschiebbares unteres Geräteteil (3),
- ein Anschlussteil für die Kartusche in dem oberen Geräteteil,
- einen Entnahmestutzen (15) in dem oberen Geräteteil zum Entnehmen der Flüssigkeit,
- eine Anstechvorrichtung auf der Innenseite des Bodens des unteren Geräteteils (3) für die auf der Unterseite des Hülsenbodens angebrachte Siegelfolie (23).

## Claims

1. A cartridge for a liquid, which can be connected to a dispensing device which includes an upper portion for receiving the cartridge and a lower portion which can be pushed on over the connected cartridge, and the upper portion of the device is provided with a connection portion for the cartridge and with a dispensing connection portion for drawing off the liquid, wherein
- the cartridge (1) is a three-shell container and comprises an outer stiff casing (4), a container (5) which is stable in respect of shape and which is disposed in the casing, and a collapsible bag (6) which is arranged in the container that is stable in respect of shape and which contains the liquid, and
- the stiff casing has a bottom provided with an opening (22), and
- the container which is stable in respect of shape is provided with an opening (14) and closed by a stopper (7), and
- the stopper is provided with an insertion connection portion (17) which forms a sealingly closing, centred guide means for the dispensing connection portion, and
- the stopper is non-releasably connected by the casing (4) to the container which is stable in respect of shape.

2. A cartridge according to claim 1 wherein
- the cartridge (1) is releasably connected to the connection portion (2) by means of a plug connection.

3. A cartridge according to claim 1 wherein
- the cartridge (1) is releasably connected to the connection portion (2) by means of a screw connection or a bayonet connection.

4. A cartridge according to claims 1 to 3 wherein
- the stopper (7), preferably of a thermoplastic material, is connected to the container (5) which is stable in respect of shape, in force-locking and positively locking relationship, by means of a snap connection (8).

5. A cartridge according to claims 1 to 3 wherein
- the stopper (7) comprising a thermoplastic material is non-releasably connected to the container (5) which is stable in respect of shape and which comprises a thermoplastic material by means of a welded join with merging of the materials.

6. A cartridge according to claims 1 to 5 wherein
- the stopper (7) is provided in the insertion connection portion (17) with a funnel-shaped centred guide means which possibly includes guide ribs (7a).

7. A cartridge according to claims 1 to 6 wherein
- the stopper (7) is provided with a press fit (17a) as sealingly closing guide means, for the dispensing connection portion.

8. A cartridge according to claims 1 to 7 wherein
- the stopper (7) is provided at the end of the insertion connection portion with a diaphragm (18) which is preferably disposed inclinedly with respect to the axis of the insertion connection portion.

9. A cartridge according to claims 1 to 4 or 6 to 8 wherein
- disposed between the upper edge of the container which is stable in respect of shape and the inside of the stopper is a sealing disc (9) which is possibly provided with sealing beads.

10. A cartridge according to claims 1 to 9 wherein
- the inside of the stopper is provided with a sealing lip (10) or with a plurality of sealing lips.

11. A cartridge according to claims 1 to 10 wherein
- the stiff casing (4) - apart from the opening thereof - is diffusion-tight.

12. A cartridge according to claims 1 to 11 wherein
- the stiff casing (4) is provided with a plurality of inwardly projecting projections (4a).

13. A cartridge according to claims 1 to 12 wherein
- the stiff casing (4) is in one piece.

14. A cartridge according to claims 1 to 12 wherein
- the stiff casing is in two pieces.

15. A cartridge according to claims 1 to 14 wherein
- the stiff casing (4) is a deep-drawn metal casing, preferably of aluminium.

16. A cartridge according to claims 1 to 14 wherein
- the stiff casing (4) comprises a plastic material, preferably a thermoplastic material.

17. A cartridge according to claims 1 to 16 wherein
- the stiff casing (4) is provided at its bottom with a projecting bead (20).

18. A cartridge according to claims 1 to 17 wherein
- the stiff casing (4) has a recess in its bottom.

19. A cartridge according to claim 18 wherein
- the stiff casing (4) is provided within the recess with an opening (22) whose diameter in the case of a circular cross-section is between 0.1 millimeter and 5 millimeters.

20. A cartridge according to claim 18 wherein
- the stiff casing (4) is provided within the recess with an insert (26) including an opening, preferably in the form of a micro-opening (27), which communicates with the opening (28) in the bottom of the stiff casing, and which in the case of a circular cross-section is of a diameter of between 10 µm and 500 µm and is of a length of between 100 µm and 500 µm.

21. A cartridge according to claims 1 to 20 wherein
- the insert (26) is provided with a filter (29) in front of the micro-opening (27).

22. A cartridge according to claims 1 to 21 wherein
- the stiff casing (4) of metal is provided with at least one peripherally extending crease (12) which embraces the stopper (7) in force-locking and positively locking relationship.

23. A cartridge according to claims 1 to 21 wherein
- the stiff casing (4) of thermoplastic material is connected to the stopper (7) by means of a welded join with merging of materials.

24. A cartridge according to claims 1 to 23 wherein
- the stiff casing (4) is provided in its upper part with a peripherally extending groove (11) which embraces the lower edge of the stopper (7).

25. A cartridge according to claims 1 to 24 wherein
- the stiff casing (4) is provided in its upper part with a flanged-over portion (13) which embraces the upper edge of the stopper (7).

26. A cartridge according to claims 1 to 25 wherein
- the cartridge is sealed in the region of the stopper (7) by a - possibly diffusion-tight - sealing foil (16).

27. A cartridge according to claims 1 to 26 wherein
- the stiff casing (4) is sealed on the outside of its bottom by a - possibly diffusion-tight - sealing foil (23).

28. A cartridge according to claims 1 to 27 wherein
- provided in the central region of the sealing foil (23) on the outside of the bottom of the stiff casing (4) is a free space (24) which is covered by the sealing foil.

29. A cartridge according to claims 1 to 28
- which can be pulled out of the dispensing device by means of a withdrawal aid (30) which can be fitted on behind the projecting bead (20).

30. A cartridge according to claim 1 wherein
- the cartridge is releasably connected with the connection portion (2) to the upper portion of the device, and
- the connection portion (2) is provided with snap hooks (19) which engage into the peripherally extending groove (11) in the upper part of the stiff casing (4) after the cartridge has been pushed into the dispensing device.

31. A cartridge according to claim 1 wherein
- the cartridge is releasably connected with the connection portion (2) to the upper portion of the device, and
- the connection portion (2) is provided with snap hooks (19) which engage into a peripherally extending groove in the stopper (7) or in the container (5) which is stable in respect of shape, after the container which is stable in respect of shape has been pushed into the dispensing device.

32. A cartridge according to claim 1 for an aqueous liquid, comprising a container (5) which is stable in respect of shape and which comprises polypropylene, with a stopper (7) of polypropylene and a collapsible bag (6) of polyethylene disposed in the container (5), wherein
- the stiff casing (4) comprises plastic material, preferably polypropylene, and
- the opening (22) in the bottom of the stiff casing (4) is a bore, and
- the stopper (7) for the container (5) which is stable in respect of shape is provided with an insertion connection portion (17) which is closed at its end by a diaphragm (18) inclined with respect to the axis of the insertion connection portion, and which forms a sealingly closing, centred guide means, in the form of a press fit, for the dispensing connection portion (15), and which is connected by a snap connection to the container which is stable in respect of shape, and
- the releasable plug connection between the cartridge (1) and the connection portion (2) of the dispensing device is in the form of a snap connection in which there are provided in the connection portion (2) of the upper portion of the dispensing device snap hooks (19) which engage into the peripherally extending groove (11) in the upper region of the cartridge (1), and
- the inside of the bottom of the push-on portion (3) of the dispensing device is provided with the resilient piercing device (25) for the sealing foil (23) on the underside of the bottom of the casing.

33. A cartridge according to claim 1 for an alcoholic liquid, comprising a container (5) of polypropylene, which is stable in respect of shape, with a stopper of polypropylene and a collapsible bag (6) of polyethylene, which is disposed in the container (5), wherein
- the stiff casing (4) comprises metal, preferably aluminium, and
- the stopper (7) which is provided with an insertion connection portion (17) for the container (5) which is stable in respect of shape is closed at its end by a diaphragm (18) which is inclined with respect to the axis of the insertion connection portion, and
- the stopper (7) is provided with a sealingly closing, centred guide means, in the form of a press fit, for the dispensing connection portion (15), and
- the stopper (7) is non-releasably connected by the casing (4) to the container (5) which is stable in respect of shape, and
- the releasable plug connection between the cartridge (1) and the connection portion (2) of the dispensing device is in the form of a snap connection in which there are provided in the connection portion (2) of the dispensing device snap hooks (19) which engage into the peripherally extending groove (11) in the upper region of the cartridge, and
- provided in the bottom of the stiff casing (4) is an insert (26) with a micro-opening (27) and the micro-opening communicates with a bore (28) in the stiff casing (4), and
- the inside of the bottom of the push-on portion (3) of the dispensing device is provided with the resilient piercing device (25) for the sealing foil (23) on the underside of the bottom of the casing.

34. A cartridge according to claims 1, 32 and 33 wherein
- the liquid contains a pharmacologically active substance.

35. A cartridge according to claims 1 and 32 to 34 for a medical liquid wherein
- the medical liquid contains one or more of the active substances Berotec (Fenoterol hydrobromide); 1-(3,5-dihydroxy-phenyl)-2-[[1-(4-hydroxy-benzyl)-ethyl]-amino]-ethanol hydrobromide), Atrovent (Ipratropium bromide), Berodual (combination of Fenoterol hydrobromide and Ipratropium bromide), Salbutamol, Salbutamol sulphate, Combivent, Oxivent (Oxitropium bromide), Ba 679 (Tiotropium bromide), BEA 2108 (di-(2-thienyl)-glycolic acid tropenol ester), Flunisolid, Budesonid and Beclomethasone.

36. Use of the cartridge according to claims 1 to 35 containing a medical liquid for producing an aerosol by means of an atomiser.

37. Use of the cartridge according to claims 1 to 35 containing a medical liquid for producing an inhalable aerosol for the treatment of illnesses

38. Use of a cartridge according to claim 1 in a dispensing device which includes an upper portion for receiving the cartridge and a lower portion (3) which can be pushed on over the connected cartridge, and the upper portion of the device is provided with a connection portion for the cartridge and with a dispensing connection portion (15) for drawing off the liquid, wherein the inside of the bottom of the lower device portion (3) is provided with a piercing device for the sealing foil (23) which is disposed on the underside of the bottom of the casing.

39. A dispensing device comprising
- a cartridge according to one of claims 1 to 35,
- an upper portion for receiving the cartridge,
- a lower portion (3) which can be pushed on over the connected cartridge,
- a connection portion for the cartridge in the upper portion of the device
- a dispensing connection portion (15) in the upper portion for drawing off the liquid,
- a piercing device at the inside of the bottom of the lower device portion (3) for the sealing foil (23) which is disposed on the underside of the bottom of the casing.

## Revendications

1. Cartouche pour un liquide qui peut être raccordée à un appareil de prélèvement qui comporte une partie d'appareil supérieure recevant la cartouche et une partie d'appareil inférieure qui peut être montée sur la cartouche raccordée, et la partie d'appareil supérieure est munie d'une partie de raccordement pour la cartouche et d'une tubulure de prélèvement pour le prélèvement du liquide, où
- la cartouche (1) est un récipient à trois enveloppes et consiste en une gaine rigide externe (4), un récipient (5) indéformable situé dans la gaine et un sachet (6) qui peut s'affaisser, situé dans le récipient indéformable, qui contient le liquide, et
- la gaine rigide a un fond qui est muni d'une ouverture (22) et
- le récipient indéformable est muni d'une ouverture (14) et est fermé par un bouchon (7), et
- le bouchon est muni d'une tubulure plongeante (17) qui forme un guide centré à fermeture étanche pour la tubulure de prélèvement, et
- le bouchon est relié de manière irréversible au récipient indéformable par la gaine (4).

2. Cartouche selon la revendication 1 où
- la cartouche (1) est reliée de manière réversible à la partie de raccordement (2) au moyen d'une liaison à enfoncement.

3. Cartouche selon la revendication 1 où
- la cartouche (1) est reliée de manière réversible à la partie de raccordement (2) au moyen d'une liaison à vis ou d'une liaison à baïonnette.

4. Cartouche selon les revendications 1 à 3 où
- le bouchon (7), constitué de préférence par une matière thermoplastique, est relié au récipient indéformable (5) par assemblage par force ou de forme au moyen d'une liaison à encliquetage (8).

5. Cartouche selon les revendications 1 à 3 où
- le bouchon (7) constitué par une matière thermoplastique est relié irréversiblement au récipient indéformable (5) constitué par une matière thermoplastique par assemblage de matière au moyen d'une liaison par soudage.

6. Cartouche selon les revendications 1 à 5 où
- le bouchon (7) est muni dans la tubulure plongeante (17) d'un guide centré en forme d'entonnoir qui contient éventuellement des nervures de guidage (7a).

7. Cartouche selon les revendications 1 à 6 où
- le bouchon (7) sous forme de guide à fermeture hermétique pour la tubulure de prélèvement est muni d'un ajustage serré (17a).

8. Cartouche selon les revendications 1 à 7 où
- le bouchon (7) est muni à l'extrémité de la tubulure plongeante d'une membrane (18) qui est disposée de préférence inclinée par rapport à l'axe de la tubulure plongeante.

9. Cartouche selon les revendications 1 à 4 ou 6 à 8 où
- entre le bord supérieur du récipient indéformable et le côté intérieur du bouchon est situé un disque d'étanchéité (9) qui est éventuellement muni de bourrelets d'étanchéité.

10. Cartouche selon les revendications 1 à 9 où
- le côté intérieur du bouchon est muni d'une lèvre d'étanchéité (10) ou de plusieurs lèvres d'étanchéité.

11. Cartouche selon les revendications 1 à 10 où
- la gaine rigide (4) est imperméable à la diffusion, sauf son ouverture.

12. Cartouche selon les revendications 1 à 11 où
- la gaine rigide (4) est munie de plusieurs saillies (4a) dirigées vers l'intérieur.

13. Cartouche selon les revendications 1 à 12 où
- la gaine rigide (4) est d'une pièce.

14. Cartouche selon les revendications 1 à 12 où
- la gaine rigide est en deux parties.

15. Cartouche selon les revendications 1 à 14 où
- la gaine rigide (4) est une gaine métallique formée par emboutissage profond, de préférence en aluminium.

16. Cartouche selon les revendications 1 à 14 où
- la gaine rigide (4) consiste en une matière plastique, de préférence en une matière thermoplastique.

17. Cartouche selon les revendications 1 à 16 où
- la gaine rigide (4) est munie en son fond d'un bourrelet en saillie (20).

18. Cartouche selon les revendications 1 à 17 où
- la gaine rigide (4) comporte un évidement en son fond.

19. Cartouche selon revendication 18 où
- la gaine rigide (4) est munie dans l'évidement d'une ouverture (22) dont le diamètre est de 0,1 millimètre à 5 millimètres dans le cas d'une section transversale circulaire.

20. Cartouche selon revendication 18 où
- la gaine rigide (4) est munie dans l'évidement d'une pièce d'insertion (26) qui contient une ouverture, de préférence sous forme d'une micro-ouverture (27), qui communique avec l'ouverture (28) au fond de la gaine rigide, et qui a un diamètre de 10 µm à 500 µm dans le cas d'une section transversale circulaire et une longueur de 100 µm à 500 µm.

21. Cartouche selon les revendications 1 à 20 où
- la pièce d'insertion (26) est munie d'un filtre (29) devant la micro-ouverture (27).

22. Cartouche selon les revendications 1 à 21 où
- la gaine rigide (4) en métal est munie d'au moins une moulure périphérique (12) qui entoure par assemblage par force et de forme le bouchon (4).

23. Cartouche selon les revendications 1 à 21 où
- la gaine rigide (4) en matière thermoplastique est reliée au bouchon (7) au moyen d'une liaison par soudage.

24. Cartouche selon les revendications 1 à 23 où
- la gaine rigide (4) est munie dans sa partie supérieure d'une rainure périphérique (11) qui entoure le bord inférieur du bouchon (7).

25. Cartouche selon les revendications 1 à 24 où
- la gaine rigide (4) est munie dans sa partie supérieure d'un bord rabattu (13) qui entoure le bord supérieur du bouchon (7).

26. Cartouche selon les revendications 1 à 25 où
- la cartouche est scellée dans le domaine du bouchon (7) avec une feuille de scellement (16) éventuellement imperméable à la diffusion.

27. Cartouche selon les revendications 1 à 26 où
- la gaine rigide (4) est scellée sur le côté extérieur de son fond avec une feuille de scellement (23) éventuellement imperméable à la diffusion.

28. Cartouche selon les revendications 1 à 27 où
- dans le domaine central de la feuille de scellement (23) sur le côté extérieur du fond de la gaine rigide (4) il est prévu un espace libre (24) recouvert par la feuille de scellement.

29. Cartouche selon les revendications 1 à 28
- qui peut être retirée de l'appareil de prélèvement au moyen d'un auxiliaire de retrait (30) qui peut être appliqué par enfoncement derrière le bourrelet en saillie (20).

30. Cartouche selon la revendication 1 où
- la cartouche est reliée de manière réversible à la partie d'appareil supérieure avec la partie de raccordement (2), et
- la partie de raccordement (2) est munie de crochets d'encliquetage (19) qui pénètrent dans la rainure périphérique (11) dans la partie supérieure de la gaine rigide (4), après que la cartouche a été insérée dans l'appareil de prélèvement.

31. Cartouche selon la revendication 1 où
- la cartouche est reliée de manière réversible à la partie d'appareil supérieure avec la partie de raccordement (2) et
- la partie de raccordement (2) est munie de crochets d'encliquetage (19) qui pénètrent dans une rainure périphérique dans le bouchon (7) ou dans le récipient indéformable (5), après que le récipient indéformable a été inséré dans l'appareil de prélèvement.

32. Cartouche selon la revendication 1 pour un liquide aqueux, consistant en un récipient indéformable (5) en polypropylène avec un bouchon (7) en polypropylène et avec un sachet (6) en polyéthylène qui peut s'affaisser, situé dans le récipient (5), où
- la gaine rigide (4) consiste en matière plastique, de préférence en polypropylène, et
- l'ouverture (22) dans le fond de la gaine rigide (4) est un alésage, et
- le bouchon (7) pour le récipient indéformable (5) est muni d'une tubulure plongeante (17) qui est fermée à son extrémité par une membrane (18) inclinée par rapport à l'axe de la tubulure plongeante, et qui forme un guide centré à fermeture étanche sous forme d'ajustage serré pour la tubulure de prélèvement (15), et qui est relié au récipient indéformable par une liaison à encliquetage, et
- la liaison à enfoncement réversible entre la cartouche (1) et la partie de raccordement (2) de l'appareil de prélèvement est sous forme de liaison à encliquetage, dans laquelle il est prévu, dans la partie de raccordement (2) de la partie d'appareil supérieure, des crochets d'encliquetage (19) qui pénètrent dans la rainure périphérique (11) dans le domaine supérieur de la cartouche (1), et
- le côté intérieur du fond de la partie d'appareil (3) qui peut être montée par emboîtement est munie du dispositif de perçage élastique (25) pour la feuille de scellement (23) sur le côté inférieur du fond de la gaine.

33. Cartouche selon la revendication 1 pour un liquide alcoolique, consistant en un récipient indéformable (5) en polypropylène avec un bouchon en polypropylène et avec un sachet (6) en polyéthylène qui peut s'affaisser, situé dans le récipient (5), où
- la gaine rigide (4) consiste en métal, de préférence en aluminium, et
- le bouchon (7) muni d'une tubulure plongeante (17) pour le récipient indéformable (5) est fermé à son extrémité par une membrane (18) inclinée par rapport à l'axe de la tubulure plongeante, et
- le bouchon (7) est muni d'un guide centré à fermeture étanche sous forme d'ajustage serré pour la tubulure de prélèvement (15), et
- le bouchon (7) est relié au récipient indéformable (5) de manière irréversible par la gaine (4), et
- la liaison à enfoncement réversible entre la cartouche (1) et la partie de raccordement (2) de l'appareil de prélèvement est sous forme de liaison à encliquetage, dans laquelle il est prévu, dans la partie de raccordement (2) de l'appareil de prélèvement, des crochets d'encliquetage (19) qui pénètrent dans la rainure périphérique (11) dans le domaine supérieur de la cartouche, et
- il est prévu dans le fond de la gaine rigide (4) une pièce d'insertion (26) avec une micro-ouverture (27), et la micro-ouverture communique avec un alésage (28) dans la gaine rigide (4), et
- le côté intérieur du fond de la partie d'appareil (3) qui peut être montée par enfoncement est munie du dispositif de perçage élastique (25) pour la feuille de scellement (23) sur le côté inférieur du fond de la gaine.

34. Cartouche selon les revendications 1, 32 et 33 où
- le liquide contient un principe actif pharmacologiquement actif.

35. Cartouche selon les revendications 1 et 32 à 34 pour un liquide médicinal, où
- le liquide médicinal contient un ou plusieurs des principes actifs berotec (bromhydrate de fénotérol ; bromhydrate de 1-(3,5-dihydroxy-phényl)-2-[[1-(4-hydroxy-benzyl)-éthyl]-amino]éthanol), atrovent (bromure d'ipratropium), berodual (combinaison de bromhydrate de fénotérol et de bromure d'ipratropium), salbutamol, sulfate de salbutamol, combivent, oxivent (bromure d'oxitropium), Ba 679 (bromure de tiotropium), BEA 2108 (di-(2-thiényl)-glycolate de tropénol), flunisolide, budésonide, beclométhasone.

36. Utilisation de la cartouche selon les revendications 1 à 35 contenant un liquide médicinal pour la production d'un aérosol au moyen d'un atomiseur.

37. Utilisation de la cartouche selon les revendications 1 à 35 contenant un liquide médicinal pour la production d'un aérosol inhalable pour le traitement de maladies.

38. Utilisation d'une cartouche selon la revendication 1 dans un récipient de prélèvement qui comprend une partie d'appareil supérieure recevant la cartouche et une partie d'appareil inférieure (3) qui peut être montée sur la cartouche raccordée et la partie d'appareil supérieure est munie d'une partie de raccordement pour la cartouche et d'une tubulure de prélèvement (15) pour prélever le liquide, où le côté intérieur du fond de la partie d'appareil inférieure (3) est muni d'un dispositif de perçage pour la feuille de scellement (23) appliquée sur le côté inférieur du fond de la gaine.

39. Appareil de prélèvement qui comprend
- une cartouche selon l'une des revendications 1 à 35,
- une partie d'appareil supérieure recevant cette cartouche,
- une partie d'appareil inférieure (3) qui peut être montée sur la cartouche raccordée,
- une partie de raccordement pour la cartouche, qui est munie à la partie d'appareil supérieure,
- une tubulure de prélèvement (15), qui est munie à la partie d'appareil supérieure, pour prélever le liquide,
- un dispositif de perçage, qui est muni au côté intérieur du fond de la partie d'appareil inférieure (3), pour la feuille de scellement (23) appliquée sur le côté inférieur du fond de la gaine.
